# EUROPÄISCHE PATENTSCHRIFT

(11) **EP 4 205 657 B1**
(45) Veröffentlichungstag und Bekanntmachung des Hinweises auf die Patenterteilung: **28.01.2026**
(21) Anmeldenummer: 21218102.8
(22) Anmeldetag: 29.12.2021
(51) Int. Cl.: A61B 6/02, A61B 6/00, A61B 6/40, A61B 6/50

(54) **VERFAHREN UND SYSTEM ZUR STEUERUNG EINES FFS-RÖNTGENSYSTEMS**
METHOD AND SYSTEM FOR CONTROLLING AN FFS IMAGING SYSTEM
PROCÉDÉ ET SYSTÈME DE COMMANDE D'UN SYSTÈME À RAYONS X FFS

(43) Veröffentlichungstag der Anmeldung: 05.07.2023
(73) Patentinhaber: Siemens Healthineers AG, 91301 Forchheim (DE)
(72) Erfinder: Radicke, Marcus, 90587 Veitsbronn (DE); Fritzler, Anja, 91052 Erlangen (DE); Kappler, Steffen, 91090 Effeltrich (DE); Lück, Ferdinand, 91052 Erlangen (DE); Nanke, Ralf, 91077 Neunkirchen am Brand (DE); Ritschl, Ludwig, 96155 Buttenheim (DE); Weber, Thomas, 91353 Hausen (DE)
(74) Vertreter: Siemens Healthineers Patent Attorneys

(56) Entgegenhaltungen:
- DE-A1- 102018 221 559
- M. KACHELRIESS ET AL: "Flying focal spot (FFS) in cone-beam CT", IEEE TRANSACTIONS ON NUCLEAR SCIENCE, vol. 53, no. 3, 1 June 2006 (2006-06-01), pages 1238 - 1247, XP055114678, ISSN: 0018-9499, DOI: 10.1109/TNS.2006.874076

## Beschreibung

Die Erfindung betrifft ein Verfahren und eine Steuereinrichtung zur Steuerung eines FFS-Röntgensystems, bevorzugt für die Tomosynthese. Insbesondere betrifft die Erfindung die Steuerung einer FFS-Ablenkung und von Kollimatorblenden eines Röntgenstrahlers.

Bei tomographischen Verfahren der Röntgendiagnostik wird in den letzten Jahren ein Verfahren eingesetzt, welches unter dem Namen "Flying Focal Spot" (FFS) bekannt ist. Bei diesem Verfahren wird der von der Kathode ausgehende Elektronenstrahl eines Röntgenstrahlers vor seinem Auftreffen auf die Anode durch ein, z.B. von einer Magnetspule erzeugtes, Magnetfeld abgelenkt. Hierdurch kann der Auftreffpunkt des Elektronenstrahls auf der Anodenoberfläche verändert werden. FFS kommt unter anderem in der Mammographie zum Einsatz.

Das Prinzip des FFS kann dazu verwendet werden, einen Röntgenstrahl gezielt über einen Bereich zu schwenken oder um eine Ortsabweichung eines Röntgenstrahls zu kompensieren. Um eine periodische Bewegung des Röntgenstrahls zu erreichen, kann die Magnetspule mit einem periodischen Ablenkstrom beaufschlagt werden. Derzeit wird häufig ein Ablenkstrom als periodische Funktion mit einer Periodenlänge von z.B. 200 ms angenommen (z.B. als Sägezahnfunktion).

Bei einem sich bewegenden Röntgenstrahler, wie er z.B. bei der Tomosynthese in der Mammographie eingesetzt wird, hat FFS insbesondere die Funktion, während der Belichtungszeit eines Objektes (z.B. 40 ms oder 70 ms) die mechanische Bewegung des Röntgenstrahlers im Raum durch eine entgegengesetzte Bewegung des Brennflecks auszugleichen, so dass dieser während einer Projektion ortsfest erscheint.

Bei einem rotierenden Röntgenstrahler kann mit oder ohne Einsatz von FFS das Problem auftreten, dass eine Überstrahlung von Randbereichen erfolgt, d.h. dass der Röntgenstrahl außer dem Detektor weitere Bereiche bestrahlt, die nicht bestrahlt werden sollen. Zwar ist der Detektor von einem Randbereich umgeben, auf den durchaus Röntgenstrahlung treffen kann, jedoch sollte keine Strahlung über diesen Bereich hinausreichen, da sie dort auf eine Person treffen kann.

Beispielsweise bewegt sich bei einer Brust-Tomosynthese der Röntgenstrahler in einem Kreisbogen über den Detektor. Die Arme der untersuchten Patientin befinden sich währenddessen in der Regel rechts und links von der Auflage, auf der die Brust untersucht wird. Reicht der Röntgenstrahl über die Ränder der Auflage (also über die Ränder des Detektors bzw. ggf. über dessen Randbereiche) hinaus, so tritt eine laterale Überstrahlung des Detektors auf und die Arme erleiden eine Exposition und die Patientin würde dort eine nachteilhafte Dosis aufnehmen.

Diesem Problem wird bei herkömmlichen Systemen mit Blenden entgegengewirkt. Diese Blenden sind häufig Kollimatorplatten und begrenzen den Strahlkegel des Röntgenstrahls auf einen vordefinierten Bereich auf dem Detektor. Bei Verwendung von FFS verschiebt sich jedoch der Fokuspunkt relativ zur Blende, was wiederum ein Überstrahlen auf unerwünschte Bereiche zur Folge haben könnte.

DE 10 2018 221 559 A1 beschreibt eine Tomosyntheseeinrichtung mit einem Röntgendetektor, einem mittels eines Bewegungsaktors relativ zu dem Röntgendetektor entlang einer Aufnahmetrajektorie bewegbaren Röntgenstrahler und einer Steuereinrichtung zur Steuerung des Betriebs der Tomosyntheseeinrichtung, wobei die Tomosyntheseeinrichtung ferner wenigstens ein Gehäuse aufweist, innerhalb dessen der Röntgenstrahler entlang der Aufnahmetrajektorie verborgen bewegbar ist.

Es ist eine Aufgabe der vorliegenden Erfindung, ein verbessertes Verfahren und ein entsprechendes System zur Steuerung eines FFS-Röntgensystems, mit dem die oben beschriebenen Nachteile vermieden werden und insbesondere eine Überstrahlung verhindert werden kann.

Diese Aufgabe wird durch ein Verfahren gemäß Patentanspruch 1, ein System gemäß Patentanspruch 11 sowie durch ein Röntgensystem gemäß Patentanspruch 12 gelöst.

Das erfindungsgemäße Verfahren dient zur Steuerung eines FFS-Röntgensystems. Ein FFS-Röntgensystem (also ein Röntgensystem mit einem "Flying Focal Spot") ist dem Fachmann im Grunde bekannt und umfasst neben einem Röntgenstrahler einen (Röntgen) Detektor zur Detektion der vom Röntgenstrahler ausgehenden Strahlung und eine FFS-Ablenkspule zum Ablenken des Elektronenstrahls im Röntgenstrahler in einer Ablenkrichtung. Der Röntgenstrahler ist bevorzugt ein FFS-Röntgenstrahler, der als funktionale Elemente eine Kathode, eine Anode und zusätzlich die FFS-Ablenkspule umfasst. Die FFS-Ablenkspule kann dabei innerhalb oder außerhalb des Vakuumgehäuses (der Röntgenröhre) des Röntgenstrahlers angeordnet sein.

Was die Ablenkrichtung umfasst, ist damit eine positive FFS-Ablenkung (in eine Richtung) und eine negative FFS-Ablenkung (in die andere Richtung) gemeint. Fließt durch die FFS-Ablenkspule ein Strom, so wird durch diesen ein Magnetfeld induziert und der Elektronenstrahl wird gemäß der Lorentzkraft abgelenkt (quer zu seiner Bewegungsrichtung). Bei einem positiven Strom erfolgt die FFS-Ablenkung in die eine Richtung, bei einem negativen Strom (umgekehrte Polarität) erfolgt die FFS-Ablenkung in die Gegenrichtung.

Der Röntgenstrahler wird dabei zum Anfertigen einer Vielzahl von Projektionsbildern in einer kreisförmigen Bewegung um den Detektor herum geführt. Zur Aufnahme der Projektionsbilder wird ein durch eine Kollimatorblende kollimierter Röntgenstrahl während der kreisförmigen Bewegung vielfach an und ausgeschaltet. Jedes Mal, wenn der Röntgenstrahler angeschaltet und dessen Röntgenstrahl auf den Detektor fällt kann ein neues Projektionsbild aufgenommen werden. Der Röntgenstrahl kann dabei im Rahmen einer FFS-Ablenkung mit der FFS-Ablenkspule beeinflusst werden, indem der Fokuspunkt des den Röntgenstrahl erzeugenden Elektronenstrahls zwischen Kathode und Anode des Röntgenstrahlers abgelenkt wird.

Das Verfahren umfasst die folgenden Schritte:
- Simulation einer Strahlgeometrie des Röntgenstrahls bei einer vorgegebenen FFS-Ablenkung auf den Detektor während einer Aufnahme eines Projektionsbildes,
- Ermittlung ob eine Überstrahlung eines vorbestimmten Bereichs um den Detektor durch den simulierten Röntgenstrahl vorliegt,
- Generieren von FFS-Steuerdaten für die Aufnahme, welche in dem Fall, dass für die Aufnahme eine Überstrahlung vorliegt, eine zur vorgegebenen FFS-Ablenkung reduzierte FFS-Ablenkung für diese Aufnahme bewirken, und ansonsten die vorgegebene FFS-Ablenkung bewirken,
- Wiederholung der Schritte für mindestens eine weitere Aufnahme,
- Generierung eines Steuerdatensatzes umfassend die generierten FFS-Steuerdaten zur Steuerung eines FFS-Röntgensystems.

Eine Strahlgeometrie lässt sich bei bekannten Positionen von Fokuspunkt, Kollimatorblende (die Blendenöffnung ist bekannt) und Detektor (dessen Maße sind bekannt) vergleichsweise einfach simulieren, da im Grunde nur ein Kegel vom Fokuspunkt aus konstruiert werden muss, der durch die Kollimatorblende begrenzt wird und ermittelt werden muss, welchen Flächenbereich der Kegel in der Ebene des Detektors überdeckt. Dieser Flächenbereich ist der vom Röntgenstrahl "bestrahlte Bereich" in der Ebene des Detektors. Letztendlich kommt es auf diesen bestrahlten Bereich an. Es sei noch hinzugefügt, dass die Kollimatorblende üblicherweise so eingestellt werden sollte, dass nur der Bereich des Detektors bestrahlt wird, auf dem auch Brustgewebe projiziert wird.

Hierzu muss jedoch beachtet werden, dass sich der Röntgenstrahler und damit auch der Fokuspunkt und die Kollimatorblende während einer Aufnahme auf einer Kreisbahn bewegen. Während der Aufnahme befinden sich diese Elemente nicht in einer festen Aufnahmeposition, sondern bewegen sich auf einer Kreisbogenbahn. Zudem bewegt sich der Fokuspunkt bei einer FFS-Ablenkung zusätzlich relativ zur Kollimatorblende. All dies muss bei der Simulation berücksichtigt werden. Mit einer "Simulation" ist im Übrigen jegliche Art der Ermittlung gemeint, die nicht einer direkten Messung entspricht. Die Strahlgeometrie kann aus geometrischen Daten berechnet werden, aber auch z.B. in einer Look-Up-Tabelle nachgeschaut werden. Es ist auch möglich, Messdaten einer vergangenen Untersuchung zu verwenden.

Da letztendlich eine Überstrahlung ermittelt werden soll, und damit der oben genannte bestrahlte Bereich ermittelt werden sollte, kann die Simulation diskrete Zeitpunkte während der Aufnahme betreffen, z.B. zum Anfang der Aufnahme und zum Ende ggf. mit einigen Berechnungen für Zeitpunkte dazwischen. Letztendlich ist es von Vorteil, wenn der maximale bestrahlte Bereich ermittelt wird, insbesondere mit Angaben, welche Dosis wo deponiert worden ist. Die Dosis kann ggf. direkt von der Intensität des Röntgenstrahls abgeleitet werden.

Da die Fläche des Detektors bekannt ist, lässt sich ermitteln, ob während einer Aufnahme der bestrahlte Bereich komplett auf dem Detektor lag oder über diesen hinausreichte. Hierzu sollte beachtet werden, dass der Detektor oftmals von einem Randbereich umgeben ist, der der Halterung und auch dem Strahlenschutz dient. Dieser Bereich ist in der Regel aus einem strahltransparenten Material gefertigt, z.B. GFK-Kunststoff. An diesem Bereich liegen oftmals direkt Teile einer Person an, z.B. die Arme einer Patientin bei einer Mammographie. Als vorbestimmter Bereich kann hier z.B. die Fläche des Detektors gewählt werden (wenn man generell eine Überstrahlung des Detektors verhindern möchte) oder die Fläche des Detektors zuzüglich des Randbereichs (wenn man lediglich eine Exposition von Körperteilen verhindern möchte).

Da der bestrahlte Bereich durch die Simulation und der vorbestimmte Bereich bekannt sind, lässt sich eine Überstrahlung einfach ermitteln, indem geschaut wird, ob der bestrahlte Bereich während einer Aufnahme stets innerhalb des vorbestimmten Bereichs liegt oder über diesen hinausreicht.

Wenn der bestrahlte Bereich zusätzlich Informationen zulässt, welche Dosis wo deponiert worden ist, kann sogar ermittelt werden, welche Dosis bei der Überstrahlung an welcher Stelle deponiert wurde.

Es wird nun entschieden, ob eine Überstrahlung vorliegt oder nicht. Bevorzugt wird dies dadurch erreicht, dass ermittelt wird, ob simulierte Röntgenstrahlen außerhalb des vorbestimmten Bereichs liegen und insbesondere zusätzlich, ob dort die Intensität durch die simulierten Röntgenstrahlen über einer vorbestimmten Schwelle liegt. Eine Schwelle kann insbesondere ein vorgegebenes Maß für eine Fläche oder eine Dosis betreffen und/oder vorgegebene Bereiche (z.B. dort wo sich Körperteile eines Menschen befinden könnten). Es kann also z.B. ermittelt werden, ob die bestrahlte Fläche, die über den vorbestimmten Bereich herausreicht, größer als ein bestimmtes Flächenmaß (ggf. auch Null) ist oder es kann ermittelt werden, ob die außerhalb des vorbestimmten Bereichs deponierte Dosis größer als ein Schwellenwert ist. Es kann aber auch ermittelt werden, ob eine bestimmte Dosis in einer bestimmten Fläche deponiert wurde (z.B. den Armen einer Patientin).

Basierend auf diesem Wissen um die Überstrahlung werden nun die FFS-Steuerdaten für den Steuerdatensatz generiert. Dieser Steuerdatensatz könnte auch als "FFS-Steuerdatensatz" bezeichnet werden, da die FFS-Steuerdaten so gestaltet sind, dass sie eine FFS-Ablenkung bewirken. Da dieser Steuerdatensatz jedoch auch weitere Steuerdaten umfassen kann, z.B. Strahl-Steuerdaten oder Blenden-Steuerdaten wie nachfolgend genauer ausgeführt wird, wird weiterhin die Bezeichnung "Steuerdatensatz" verwendet.

Normalerweise sind die FFS-Steuerdaten für eine Aufnahme so gestaltet, dass sie die vorgegebene FFS-Ablenkung bewirken. Diese kann insbesondere für jede Aufnahme (eines Projektionsbildes) gleich sein, z.B. dass der Fokuspunkt während einer Aufnahme relativ zum Detektor ruht.

Gemäß dem erfindungsgemäßen Verfahren werden aber nun zwei Fälle unterschieden, nämlich der, dass eine Überstrahlung vorliegt oder nicht. Dass eine Überstrahlung vorliegt, kann (wie oben gesagt) insbesondere anhand einer vorbestimmten Schwelle festgelegt werden.

Liegt eine bestimmte Überstrahlung vor, werden für die betreffende Aufnahme (eines Projektionsbildes) FFS-Steuerdaten für eine zur vorgegebenen FFS-Ablenkung reduzierte FFS-Ablenkung generiert, wenn nicht FFS-Steuerdaten für die vorgegebene FFS-Ablenkung.

Dies wird dann für mindestens eine weitere Aufnahme (eines Projektionsbildes), insbesondere für die Aufnahme aller Projektionsbilder, wiederholt. Dabei ist bevorzugt, die Berechnungen zumindest für große Winkel relativ zur Senkrechten über dem Detektor durchzuführen, da dort die Gefahr einer Überstrahlung am größten ist.

Dann wird der Steuerdatensatz aus den generierten FFS-Steuerdaten (vorgegebene und ggf. reduzierte) zur Steuerung eines FFS-Röntgensystems erstellt. Dieser kann direkt zur Steuerung des FFS-Röntgensystems verwendet werden, oder aber abgespeichert und zur Steuerung eines FFS-Röntgensystems mit ähnlichem oder identischen Aufbau verwendet werden. Hierzu sollte jedoch beachtet werden, dass zuweilen die Kollimatorblende für jeden Patienten individuell eingestellt wird (bei einer Mammographie kann die Kollimatoreinstellung von der aktuell Brustgröße abhängen) oder unterschiedliche Belichtungszeiten pro Projektionsbild vorliegen (je länger die Belichtungszeit desto größer die maximale Auslenkung des FFS), so dass das Verfahren bevorzugt vor einer Untersuchung nach Einstellen der Kollimatorblende bzw. Wahl der Belichtungszeit durchgeführt wird.

Ein mittels dieses Verfahrens erstellter Steuerdatensatz ist ebenfalls Teil der Erfindung. Beispielsweise könnte das Verfahren für mehrere mögliche Blendenöffnungen durchgeführt werden und der daraus resultierende Datensatz für FFS-Röntgensysteme verwendet werden, wobei nach Einstellung der Kollimatorblende einfach die entsprechenden Steuerdaten gemäß der Einstellung der Kollimatorblende ausgewählt werden.

Ein erfindungsgemäßes System dient (wie das Verfahren) zur Steuerung eines FFS-Röntgensystems mit einem Röntgenstrahler, welcher zum Anfertigen einer Vielzahl von Projektionsbildern in einer kreisförmigen Bewegung um einen Detektor herum geführt wird und ein durch eine Kollimatorblende kollimierter Röntgenstrahl während der Bewegung vielfach an und ausgeschaltet wird, und wobei das FFS-Röntgensystem für eine FFS-Ablenkung eine FFS-Ablenkspule umfasst, mit der ein Fokuspunkt eines den Röntgenstrahl erzeugender Elektronenstrahls zwischen Kathode und Anode des Röntgenstrahlers abgelenkt werden kann. Das System umfasst die folgenden Komponenten:
- eine Simulationseinheit ausgelegt zur Simulation einer Strahlgeometrie des Röntgenstrahls bei einer vorgegebenen FFS-Ablenkung auf den Detektor während einer Aufnahme eines Projektionsbildes,
- eine Ermittlungseinheit ausgelegt zur Ermittlung ob eine Überstrahlung eines vorbestimmten Bereichs um den Detektor durch den simulierten Röntgenstrahl vorliegt,
- eine Steuerdateneinheit ausgelegt zur Generieren eines erfindungsgemäßen Steuerdatensatzes.

Die Arbeitsweise der Einheiten wurde im Rahmen des Verfahrens bereits ausführlich beschrieben. Zur Bewegung einer Kollimatorblende kann das System noch eine spezielle Bewegungseinheit aufweisen. Ein FFS-Röntgensystem, welches eine automatisiert bewegliche Kollimatorblende aufweist, verfügt jedoch in der Regel über eine Bewegungseinheit, z.B. ein Elektromotor mit Getriebe, die einfach mit Steuersignalen angesteuert werden kann.

Ein erfindungsgemäßer FFS-Röntgenstrahler umfasst eine Steuereinrichtung und ein erfindungsgemäßes System, welches durchaus in der Steuereinrichtung vorliegen kann. Alternativ oder zusätzlich kann der FFS-Röntgenstrahler, bzw. dessen Steuereinrichtung, auch einen erfindungsgemäßen Steuerdatensatz umfassen, z.B. in einer Speichereinheit.

Eine erfindungsgemäße Steuereinrichtung für ein Röntgensystem, umfasst ein erfindungsgemäßes System oder einen erfindungsgemäßen Steuerdatensatz.

Ein Großteil der zuvor genannten Komponenten des Systems bzw. der Steuereinrichtung, können ganz oder teilweise in Form von Softwaremodulen in einem Prozessor eines entsprechenden Rechensystems realisiert werden. Eine weitgehend softwaremäßige Realisierung hat den Vorteil, dass auch schon bisher verwendete Rechensysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem ladbar ist, mit Programmabschnitten, um alle Schritte des erfindungsgemäßen Verfahrens auszuführen, wenn das Programm in dem Rechensystem ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem bzw. zur Steuereinrichtung und/oder zur Speicherung an oder in dem Rechensystem bzw. der Steuereinrichtung kann ein computerlesbares Medium, z.B. ein Memorystick, eine Festplatte oder ein sonstiger transportabler oder fest eingebauter Datenträger dienen, auf welchem die von einem Rechensystem bzw. einer Rechnereinheit der Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Gemäß einem bevorzugten Verfahren erfolgt eine Reduzierung der FFS-Ablenkung für eine Aufnahme (eines Projektionsbildes) dermaßen, dass für die betreffenden Aufnahmen die FFS-Ablenkung auf Null reduziert wird. Die FFS-Steuerdaten sind also bevorzugt so gestaltet, dass für diese Aufnahme keine FFS-Ablenkung erfolgt.

Gemäß einem bevorzugten Verfahren kann aber auch einfach keine Aufnahme (eines Projektionsbildes) erfolgen. Der Steuerdatensatz kann also hier Strahl-Steuerdaten umfassen, welche einfach den Strahl für die betreffende Aufnahmeposition nicht einschalten und damit auch keine Aufnahme (eines Projektionsbildes) erfolgt. Theoretisch können hierzu in dem Schritt des Verfahrens, in dem FFS-Steuerdaten generiert werden, zusätzlich oder alternativ zu den FFS-Steuerdaten Strahl-Steuerdaten für die betreffende Aufnahme(n) generiert werden, welche in dem Fall, dass für die Aufnahme (eines Projektionsbildes) eine Überstrahlung vorliegt, eine zu einer vorgegebenen Intensität des Röntgenstrahls reduzierte Intensität (kann auch Null sein) für diese Aufnahme bewirken, und ansonsten die vorgegebene Intensität.

Gemäß einem bevorzugten Verfahren weist die vorgegebenen FFS-Ablenkung einen FFS-Startpunkt und einen FFS-Endpunkt auf, zwischen denen der Fokuspunkt bei der FFS-Bewegung bewegt wird. Vor einer Aufnahme "springt" der Fokuspunkt zum FFS-Startpunkt und bewegt sich dann kontinuierlich zum FFS-Endpunkt, insbesondere wobei der Fokuspunkt im Raum (relativ zum Detektor) ruht.

Zur Reduzierung der FFS-Ablenkung wird bei der reduzierten FFS-Ablenkung der FFS-Startpunkt nun bevorzugt näher zum FFS-Endpunkt verschoben und/oder der FFS-Endpunkt näher zum FFS-Startpunkt. Der vom Fokuspunkt auf der Anode zurückgelegt Weg verkürzt sich also. Da eine Überstrahlung häufig im Bereich des normalen FFS-Startpunkts oder FFS-Endpunkts auftritt, wird durch Verschiebung dieser Punkte aufeinander zu dieses Problem beseitigt. Diese Verkürzung kann auf zwei Arten für eine FFS-Ablenkung genutzt werden.

Zum einen ist die Bewegungszeit des Fokuspunkts zwischen FFS-Startpunkt und FFS-Endpunkt bei der reduzierten FFS-Ablenkung bevorzugt gleich der vorgegebenen FFS-Ablenkung. Da die Strecke kürzer ist, wird also der Fokuspunkt langsamer geführt. Dadurch wird eine kleinere "Verschmierung" des Röntgenstrahls auf dem Detektor bei einer effektiven Unterdrückung einer Überstrahlung erreicht.

Alternativ ist die Geschwindigkeit des Fokuspunkts zwischen FFS-Startpunkt und FFS-Endpunkt bei der reduzierten FFS-Ablenkung bevorzugt gleich der vorgegebenen FFS-Ablenkung und eine FFS-Ablenkung erfolgt erst nach einer vorgegebenen Wartezeit nach Beginn der betreffenden Aufnahme (eines Projektionsbildes) und/oder mit einer Pausezeit zum Ende der Aufnahme. Der Fokuspunkt bewegt sich also beim Vorliegen einer Wartezeit und einer Pausezeit während der Wartezeit zunächst auf der Kreisbahn mit, ruht dann bei seiner FFS-Ablenkung im Raum und bewegt sich dann während der Pausezeit erneut auf der Kreisbahn. Bei dieser Alternative ist auch bevorzugt, dass der Röntgenstrahl während der Wartezeit und/oder der Pausezeit ausgeschaltet ist. Dies resultiert zwar in einer kürzeren Aufnahmezeit, hat jedoch den Vorteil, dass die aufgenommenen Bilder optimal Bewegungskompensiert sind.

Bevorzugt entspricht dabei der FFS-Startpunkt bei einem Winkel zur einen Seite (z.B. rechts) der Senkrechten über dem Detektor im Wesentlichen dem FFS-Endpunkt bei dem entsprechenden Winkel auf der anderen Seite (z.B. links) der Senkrechten und umgekehrt.

Gemäß einem bevorzugten Verfahren werden in dem Fall, dass eine Überstrahlung vorliegt, Strahl-Steuerdaten zur Reduzierung der Intensität des Röntgenstrahls (also dessen applizierte Dosis) für diese Aufnahme (eines Projektionsbildes) dem Steuerdatensatz hinzugefügt. Die Intensität wird dazu bezüglich der für eine Aufnahme verwendeten (vorbekannten) Intensität reduziert. Hierzu ist zu beachten, dass eine Überstrahlung zumeist bei relativ großen Winkeln zur Senkrechten über dem Detektor auftritt. Wenn eine FFS-Ablenkung dort reduziert ist, dann ist die Aufnahmequalität dort auch nicht optimal, da der Strahl wegen der Bewegung des Röntgenstrahlers "verschmiert". Wird nun für diese Bilder die Intensität reduziert, dann wird zum einen weniger Dosis in den überstrahlten Bereich appliziert und zum anderen kann damit die Kalksichtbarkeit verbessert werden. Es hat also durchaus Vorteile, die applizierte Dosis bei den äußeren Projektionen (große Winkel) zu verringern und bei den mittleren Projektionen zu vergrößern (bzw. nicht zu reduzieren).

Gemäß einem bevorzugten Verfahren umfasst der Steuerdatensatz zusätzlich Blenden-Steuerdaten zum Nachführen der Kollimatorblende des Röntgenstrahlers (während einer Aufnahme) entsprechend der FFS-Ablenkung des Fokuspunkts. Diese Blenden-Steuerdaten sind bevorzugt dazu ausgelegt, die Kollimatorblende in Form von zwei Bewegungen zu bewegen:
- eine Rückführungsbewegung in Richtung der kreisförmigen Bewegung des Röntgenstrahlers bei ausgeschaltetem Röntgenstrahl, wobei die Kollimatorblende nach dem Ausschalten des Röntgenstrahls an eine Startposition relativ zum Röntgenstrahler bewegt wird,
- eine kontinuierliche Nachführung gegen die Richtung der kreisförmigen Bewegung des Röntgenstrahlers bei angeschaltetem Röntgenstrahl, wobei die Kollimatorblende von der Startposition aus bis zu einer Endposition nachgeführt wird, bevorzugt wobei die Kollimatorblende so nachgeführt wird, dass sie im Wesentlichen eine konstante Position zu einer Linie zwischen Fokuspunkt und einem vorbestimmten Punkt auf dem Detektor hat, bevorzugt zum Mittelpunkt des Detektors.

Vor der Aufnahme (eines Projektionsbildes) "springt" die Kollimatorblende also an die Startposition, wird dann während der FFS-Ablenkung kontinuierlich nachgeführt, z.B. so dass sie zusammen mit dem Fokuspunkt im Raum ruht und springt danach wieder an die neue Startposition. Dies hat den Vorteil, dass durch diese Bewegung der Kollimatorblende eine Überstrahlung besser verhindert werden kann, da der Strahlkegel vom Fokuspunkt besser auf den Detektor gerichtet werden kann.

Theoretisch können in dem Schritt des Verfahrens, in dem FFS-Steuerdaten generiert werden, zusätzlich oder alternativ zu den FFS-Steuerdaten Blenden-Steuerdaten für die betreffende Aufnahme(n) generiert werden, welche in dem Fall, dass für die Aufnahme (eines Projektionsbildes) eine Überstrahlung vorliegt, eine Bewegung der Kollimatorblende erreichen können. Die Bewegung ist dergestalt, dass die Kollimatorblende einen Weg beschreibt, in der eine simulierte Strahlgeometrie keine Überstrahlung mehr aufweist bzw. die Überstrahlung unter einem Schwellenwert liegt.

Gemäß einem bevorzugten Verfahren wird die Kollimatorblende während ihrer kontinuierlichen Nachführung so bewegt, dass sie während der Bewegung des Röntgenstrahlers relativ
- zum Detektor und/oder
- zu einem Fokuspunkt und/oder
- zu einem Schnittpunkt einer Linie zwischen Fokuspunkt und einem vorbestimmten Punkt auf dem Detektor mit einer Ebene der Kollimatorblende
ruht oder sich langsamer als die Bahngeschwindigkeit der kreisförmigen Bewegung in Richtung oder entgegen der kreisförmigen Bewegung bewegt oder sich zeitversetzt zu der FFS-Ablenkung bewegt.

Diesbezüglich kommt es für ein optimales Ergebnis etwas auf die Relativbewegung von Fokuspunkt und Detektor an.

In dem Falle, in dem die vorgegebene FFS-Ablenkung bei einer Aufnahme (eines Projektionsbildes) erfolgt, ruht normalerweise der Fokuspunkt relativ zum Detektor im Raum. Die Kollimatorblende sollte für ein optimales Ergebnis hier auch im Raum ruhen (wie auch der Schnittpunkt). In dem Falle, in dem jedoch eine reduzierte FFS-Ablenkung erfolgt, kann sich der Fokuspunkt zunächst im Raum bewegen (an seinem FFS-Startpunkt) dann ruhen (während der reduzierten FFS-Bewegung) und dann wieder bewegen (an seinem FFS-Endpunkt), oder er wird mit einer reduzierten Strecke nachgeführt und bewegt sich somit im Raum langsamer als die Kreisbewegung. In diesem Falle sollte sich die Kollimatorblende bevorzugt auch im Raum bewegen und zwar relativ zum Fokuspunkt oder besser relativ zum Schnittpunkt.

Es kann aber auch eine bewusst nicht-synchrone Bewegung der Kollimatorblende mit dem Fokuspunkt erfolgen, wobei die Kollimatorblende mit einer anderen Geschwindigkeit verfahren wird als der Fokuspunkt (langsamer oder schneller) oder zeitversetzt verfahren wird, um damit nur einen Teil der Überstrahlung auszugleichen.

Gemäß einem bevorzugten Verfahren wird die Kollimatorblende nur dann kontinuierlich nachgeführt, wenn eine FFS-Ablenkung erfolgt, insbesondere nur dann, wenn ein Strom durch die FFS-Ablenkspule fließt und/oder (nur dann) wenn der Röntgenstrahler angeschaltet ist.

Gemäß einem bevorzugten Verfahren erfolgt eine Änderung der Blendenöffnung der Kollimatorblende bei Aufnahmen unterschiedlicher Projektionsbilder, bevorzugt wobei die Blendenöffnung kleiner ist, je weiter der Winkel des Röntgenstrahlers von der Senkrechten zum Detektor abweicht. Diese Änderung der Blendenöffnung kann mit oder ohne einer Nachführung der Kollimatorblende erfolgen und hat den Vorteil, dass eine Überstrahlung mit einer kleineren Blendenöffnung bei großen Winkeln des Fokuspunkts zur Senkrechten über dem Detektor besser verhindert werden kann.

Ein bevorzugtes Verfahren umfasst die zusätzlichen Schritte:
- Anfertigen einer Vielzahl von Projektionsbildern, wobei der Röntgenstrahl des Röntgenstrahlers während dessen kreisförmiger Bewegung vielfach an und ausgeschaltet wird,
- FFS-Ablenkung des Fokuspunkts während der Anfertigung der Projektionsaufnahmen gemäß dem Steuerdatensatz, bevorzugt wobei der Fokuspunkt im Falle einer FFS-Ablenkung entgegen der kreisförmigen Bewegung des Röntgenstrahlers geführt wird.

Gemäß einem bevorzugten Verfahren erfolgt nach dem Anfertigen der Projektionsbilder eine Rekonstruktion einer Anzahl von Bildern (z.B. Schichtbildern) aus den aufgenommenen Projektionsbildern. Bei der Rekonstruktion werden bevorzugt Daten von Projektionsbildern basierend auf dem Steuerdatensatz (ortsfrequenzabhängig) unterschiedlich gewichtet. Dabei ist insbesondere eine Wichtung umso geringer je größer die Reduzierung der FFS-Ablenkung war.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine grob schematische Darstellung eines bevorzugten Tomosynthesesystems mit einem bevorzugten System,
Figur 2 ein Beispiel für einen Röntgenstrahler mit einem erfindungsgemäßen System,
Figur 3 ein Beispiel für eine FFS-Ablenkung,
Figur 4 ein Beispiel für eine Aufnahme ohne FFS-Ablenkung gemäß dem Stand der Technik,
Figur 5 ein Beispiel für eine Aufnahme mit FFS-Ablenkung,
Figur 6 ein Ablaufplan für einen möglichen Ablauf eines erfindungsgemäßen Verfahrens,

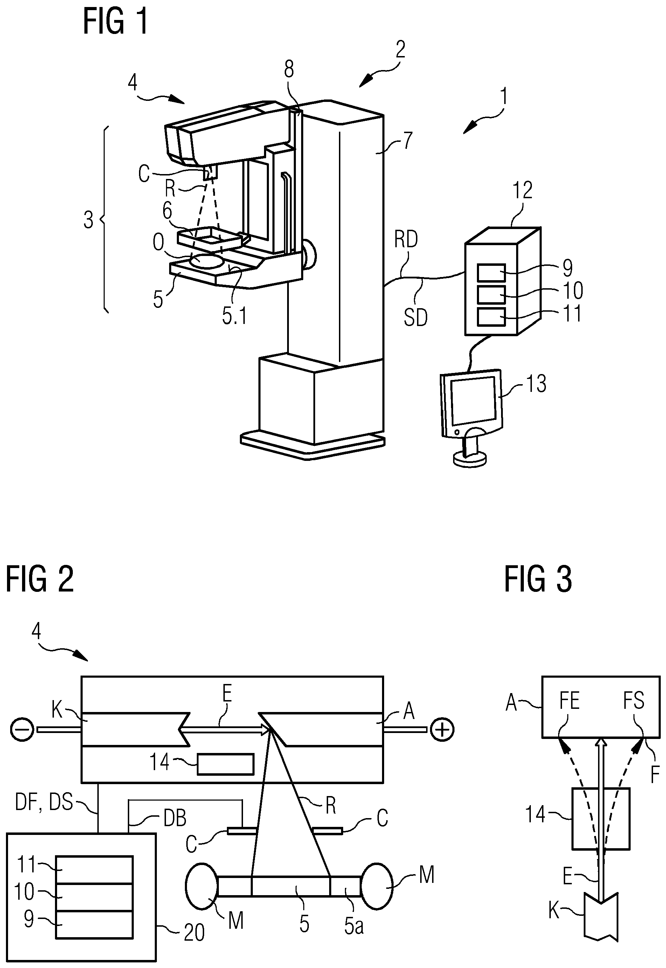

In Figur 1 ist beispielhaft und grob schematisch ein Tomosynthesesystem 1 gezeigt. Relative Richtungsangaben wie "oben", "unten" etc. beziehen sich auf ein bestimmungsgemäß für den Betrieb aufgestelltes Tomosynthesesystem 1. Das Tomosynthesesystem 1 umfasst ein Tomosynthesegerät 2 und eine Steuereinrichtung 12. Das Tomosynthesegerät 2 weist eine Standsäule 7 und eine Quelle-Detektor-Anordnung 3 auf, die wiederum eine Röntgenstrahler 4 und einen Detektor 5 mit einer Detektorfläche 5.1 umfassen. Die Standsäule 7 steht im Betrieb auf dem Untergrund. Mit ihr ist die Quelle-Detektor-Anordnung 3 verschiebbar verbunden, sodass die Höhe der Detektorfläche 5.1, also der Abstand zum Untergrund, auf eine Brusthöhe einer Patientin eingestellt werden kann.

Eine Brust O der Patientin (hier schematisch dargestellt) liegt als Untersuchungsobjekt O für eine Untersuchung oberseitig auf der Detektorfläche 5.1 auf. Über der Brust O und der Detektorfläche 5.1 ist eine Platte 6 angeordnet, die verschiebbar mit der Quelle-Detektor-Anordnung 3 verbunden ist. Für die Untersuchung wird die Brust O komprimiert und zugleich fixiert, indem die Platte 6 auf sie herabgesenkt wird, sodass auf die Brust O zwischen Platte 6 und Detektorfläche 5.1 ein Druck ausgeübt wird.

Der Röntgenstrahler 4 ist dem Detektor 5 gegenüberliegend so angeordnet und ausgebildet, dass der Detektor 5 von ihm emittierte Röntgenstrahlung R erfasst, nachdem zumindest ein Teil der Röntgenstrahlung R die Brust O der Patientin durchdrungen hat. Dabei ist der Röntgenstrahler 4 relativ zum Detektor 5 mittels eines Dreharms 8 in einem Bereich von ± 50° um eine Grundstellung schwenkbar, in der sie senkrecht über der Detektorfläche 5.1 steht.

Die Steuereinrichtung 12 erhält die Rohdaten RD der Messung und sendet Steuerdaten SD an das Tomosynthesegerät 2. Sie ist mit einem Terminal 13 verbunden, über den ein Benutzer dem Tomosynthesesystem 1 Befehle mitteilen oder Messergebnisse abrufen kann. Die Steuereinrichtung 12 kann in demselben Raum wie das Tomosynthesegerät 2 angeordnet sein, es kann sich aber auch in einem angrenzenden Kontrollraum oder in einer noch weiteren räumlichen Entfernung befinden.

Das erfindungsgemäße System 20 (s. auch Figur 2) umfasst eine Simulationseinheit 9, eine Ermittlungseinheit 10 und eine Steuerdateneinheit 11.

Die Simulationseinheit 9 ist zur Simulation einer Strahlgeometrie des Röntgenstrahls R bei einer vorgegebenen FFS-Ablenkung auf den Detektor 5 während einer Aufnahme eines Projektionsbildes P ausgelegt.

Die Ermittlungseinheit 10 ist zu einer Ermittlung ausgelegt, ob eine Überstrahlung eines vorbestimmten Bereichs um den Detektor 5 durch den simulierten Röntgenstrahl R vorliegt.

Die Steuerdateneinheit 11 ist zur Generieren eines Steuerdatensatzes D ausgelegt. Sie ist diesbezüglich in der Lage, FFS-Steuerdaten DF und bevorzugt auch Strahl-Steuerdaten DS und Blenden-Steuerdaten DB (s. Figur 2) für die Aufnahme zu generieren. In dem Fall, dass für die Aufnahme (eines Projektionsbildes P) eine Überstrahlung vorliegt, umfasst der Steuerdatensatz D FFS-Steuerdaten DF die eine für eine zu einer vorgegebenen FFS-Ablenkung reduzierten FFS-Ablenkung für diese Aufnahme bewirken, und ansonsten FFS-Steuerdaten DF, die eine vorgegebene FFS-Ablenkung bewirken.

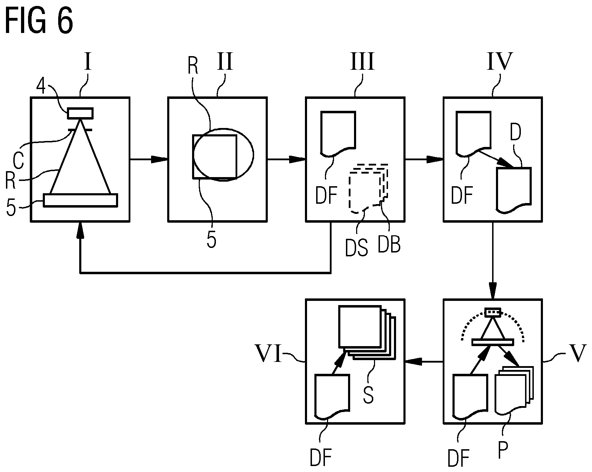

Näheres zur Funktion der Einheiten ist auch unten zu Figur 6 ausgeführt.

Figur 2 zeigt eine schematische Darstellung eines Röntgenstrahlers 4. In einem luftleeren Gehäuse (der eigentlichen Röntgenröhre) sind eine Kathode K und eine Anode A angeordnet, zwischen denen im Betrieb des Röntgenstrahlers 4 ein Elektronenstrahl E beschleunigt wird und auf die Anode A trifft.

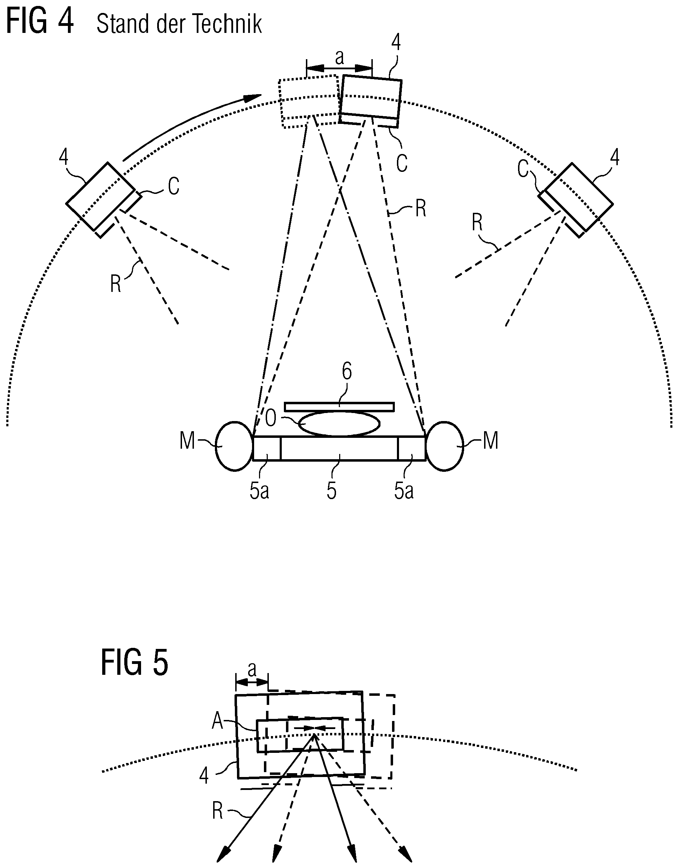

Damit der Auftreffpunkt des Elektronenstrahls E auf der Anode A verändert werden kann, ist zwischen Kathode K und Anode A eine FFS-Ablenkspule 14 angeordnet, deren Wirkung eine FFS-Ablenkung des Elektronenstrahls E ist. Wird sie mit einem Ablenkstrom beaufschlagt, so erzeugt sie ein Magnetfeld, in dem der Elektronenstrahl E abgelenkt wird. Die Ablenkung erfolgt hier abhängig von der Polarität des Ablenkstroms entweder in die Bildebene hinein oder aus ihr heraus. Während der Röntgenstrahler auf einer Kreisbahn bewegt wird (s. z.B. Figur 5), und ein Röntgenstrahl R in eine Richtung abgestrahlt wird, kann der Elektronenstrahl E mit der FFS-Ablenkspule 14 abgelenkt werden.

Der Röntgenstrahler 4 strahlt einen Röntgenstrahl R durch eine Kollimatorblende C auf einen flächigen Röntgendetektor 5 mit Randbereichen 5a an denen zwei Arme als Körperteile M eines Menschen M dargestellt sind, wobei der Elektronenstrahl E in dem Röntgenstrahler 4 mittels der FFS-Ablenkspule 14 abgelenkt werden kann.

Die FFS-Ablenkung im Röntgenstrahler 4 wird mit einem erfindungsgemäßen System 20 erreicht, wie es bereits oben genauer beschrieben wurde (s. Figur 1). Diese Ablenkung erfolgt in die Bildebene hinein und aus der Bildebene hinaus.

Von dem System 20 werden hier sowohl FFS-Steuerdaten DF als auch Strahl-Steuerdaten DS und Blenden-Steuerdaten DB gesendet, wobei die FFS-Steuerdaten DF auf die FFS-Ablenkung, die Strahl-Steuerdaten DS auf die Strahlintensität der Röntgenstrahls R und die Blenden-Steuerdaten DB auf eine Bewegung der Kollimatorblende C wirken.

Die Aufsicht in Figur 3 stellt die Ablenkung des Elektronenstrahls E in diesem Röntgenstrahler 4 dar.

Figur 4 zeigt die Bewegung eines Röntgenstrahlers 4 eines FFS-Röntgensystems 1 nach Figur 1 während einer Tomosyntheseuntersuchung gemäß dem Stand der Technik. Der Röntgenstrahler 4 bewegt sich während der Untersuchung vom Dreharm 8 geführt auf einem Kreisbogen kontinuierlich entlang des Pfeils und strahlt mit einem Röntgenstrahl R, welche durch eine Kollimatorblende C kollimiert wird, auf das zu untersuchende Objekt O auf dem Detektor 5. Währenddessen wird während dieser kontinuierlichen Bewegung eine Mehrzahl von Röntgenprojektionen oder "Röntgenschüssen" mit dem Detektor 5 aufgenommen.

Wie an der mittleren Position angedeutet bewegt sich der Röntgenstrahler 4 während einer Röntgenprojektion, die eine gewisse Zeit dauert, entlang einer Strecke a. Hier ist die zentrale Position während der Röntgenprojektion durchgezogen dargestellt und Anfangs- und Endposition gepunktet (zwischen Anfangs und Endposition erfolgt die Röntgenprojektion). Bei einer Aufnahme (eines Projektionsbildes P) bewegt sich damit der Winkel des Röntgenstrahls R, was zu einer Verschmierung des aufgenommenen Bildes führt.

Dieses Bewegungsartefakt wird durch eine FFS-Nachführung vermieden, die den Fokuspunkt stets in der zentralen Position (durchgezogen angedeutet) hält.

Figur 5 zeigt ein Beispiel für eine FFS-Nachführung während einer Tomosyntheseuntersuchung. Dargestellt ist wieder der oberste Punkt der Kreisbahn mit der Anfangsposition (hier durchgezogen) und der Endposition (punktiert) des Röntgenstrahlers 4, in dem hier noch die Anode A angedeutet ist.

Durch eine FFS-Ablenkung wird der Elektronenstrahl E, der hier von oben in die Bildebene auf die Anode A trifft, zunächst nach rechts abgelenkt und später nach links abgelenkt (Pfeile), so dass der Auftreffpunkt auf die Anode A (Fokuspunkt) über die gesamte Bewegung des Röntgenstrahlers 4 während dieser Röntgenprojektion stets an einer Position im Raum verharrt.

Figur 6 zeigt ein Ablaufplan für einen möglichen Ablauf eines erfindungsgemäßen Verfahrens, zur Steuerung eines FFS-Röntgensystems 1 wie es z.B. in Figur 1 gezeigt ist. Es wird in diesem Beispiel dabei nicht nur ein Steuerdatensatz D generiert, der alleine bereits eine Steuerung ermöglicht, sondern auch ein Bild aufgenommen und rekonstruiert.

In Schritt I erfolgt zunächst eine Simulation einer Strahlgeometrie des Röntgenstrahls R bei einer vorgegebenen FFS-Ablenkung auf den Detektor 5 während einer Aufnahme eines Projektionsbildes P. Das Projektionsbild muss dabei nicht real aufgenommen werden, da alle notwendigen Informationen vorhanden sind.

In Schritt II erfolgt eine Ermittlung ob eine Überstrahlung eines vorbestimmten Bereichs um den Detektor 5 durch den simulierten Röntgenstrahl R vorliegt. Gezeigt ist hier der Detektor 5 von oben (Viereck) und ein bestrahlter Bereich (Kreis) auf dem Detektor 5. Wie man erkennen kann reicht der Kreis über die Ränder des Detektors hinaus, was einer Überstrahlung entspricht.

In Schritt III erfolgt ein Generieren von FFS-Steuerdaten DF für die Aufnahme (eines Projektionsbildes P), welche in dem Fall, dass für die Aufnahme eine Überstrahlung vorliegt, eine zur vorgegebenen FFS-Ablenkung reduzierte FFS-Ablenkung für diese Aufnahme bewirken, und ansonsten die vorgegebene FFS-Ablenkung bewirken.

Gestrichelt ist angedeutet, dass zusätzlich noch Blenden-Steuerdaten DB zur Nachführung einer Kollimatorblende C und/oder Strahl-Steuerdaten DS zur Regelung der Intensität des Röntgenstrahls R generiert werden können.

Diese Schritte werden nun für alle Aufnahmen (Positionen des Röntgenstrahlers 4 währen der Aufnahmen) wiederholt.

In Schritt IV erfolgt die Generierung eines Steuerdatensatzes D umfassend die generierten FFS-Steuerdaten DF und ggf. zusätzlich Blenden-Steuerdaten DB und/oder Strahl-Steuerdaten DS zur Steuerung eines FFS-Röntgensystems 1.

In Schritt V erfolgt ein Anfertigen (Aufnehmen) einer Vielzahl von Projektionsbildern P, wobei der Röntgenstrahl R des Röntgenstrahlers 4 während dessen kreisförmiger Bewegung vielfach an und ausgeschaltet wird.

Währenddessen erfolgt eine FFS-Ablenkung des Fokuspunkts F gemäß dem Steuerdatensatz D, wobei der Fokuspunkt F im Falle ggf. teilweise im Raum ruht.

In Schritt VI erfolgt ein eine Rekonstruktion von Schichtbildern S aus den aufgenommenen Projektionsbildern P, wobei bei der Rekonstruktion Daten von Projektionsbildern P basierend auf dem Steuerdatensatz D unterschiedlich gewichtet werden, wobei eine Wichtung umso geringer ist, je größer die Reduzierung der FFS-Ablenkung war.

Es wird abschließend noch einmal darauf hingewiesen, dass es sich bei den vorhergehend detailliert beschriebenen Verfahren sowie bei dem dargestellten System lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen Begriffe wie "Einheit" nicht aus, dass die betreffenden Komponenten aus mehreren zusammenwirkenden TeilKomponenten bestehen, die gegebenenfalls auch räumlich verteilt sein können. Der Ausdruck "eine Anzahl" ist als "mindestens eins" zu verstehen.

## Patentansprüche

1. Verfahren zur Steuerung eines FFS-Röntgensystems (1) mit einem Röntgenstrahler (4), welcher zum Anfertigen einer Vielzahl von Projektionsbildern (P) in einer kreisförmigen Bewegung um einen Detektor (5) herum geführt wird und ein durch eine Kollimatorblende (C) kollimierter Röntgenstrahl (R) während der Bewegung vielfach an und ausgeschaltet wird, und wobei das FFS-Röntgensystem (1) für eine FFS-Ablenkung eine FFS-Ablenkspule (14) umfasst, mit der ein Fokuspunkt (F) eines den Röntgenstrahl (R) erzeugender Elektronenstrahls (E) zwischen Kathode (K) und Anode (A) des Röntgenstrahlers (4) abgelenkt werden kann, das Verfahren umfassend die Schritte:
- Simulation einer Strahlgeometrie des Röntgenstrahls (R) bei einer vorgegebenen FFS-Ablenkung auf den Detektor (5) während einer Aufnahme eines Projektionsbildes (P),
- Ermittlung, ob eine Überstrahlung eines vorbestimmten Bereichs um den Detektor (5) durch den simulierten Röntgenstrahl (R) vorliegt,
- Generieren von FFS-Steuerdaten (DF) für die Aufnahme, welche in dem Fall, dass für die Aufnahme eine Überstrahlung vorliegt, eine zur vorgegebenen FFS-Ablenkung reduzierte FFS-Ablenkung für diese Aufnahme bewirken, und ansonsten die vorgegebene FFS-Ablenkung bewirken,
- Wiederholung der Schritte für mindestens eine weitere Aufnahme,
- Generierung eines Steuerdatensatzes (D) umfassend die generierten FFS-Steuerdaten (DF) zur Steuerung eines FFS-Röntgensystems (1).

2. Verfahren nach Anspruch 1, wobei eine Reduzierung der FFS-Ablenkung für eine Aufnahme dermaßen erfolgt, dass für die betreffenden Aufnahmen die FFS-Ablenkung auf Null reduziert wird oder keine Aufnahme eines Projektionsbildes (P) erfolgt.

3. Verfahren nach einem der vorangehenden Ansprüche, wobei die vorgegebenen FFS-Ablenkung einen FFS-Startpunkt (FS) und einen FFS-Endpunkt (FE) aufweist, zwischen denen der Fokuspunkt (F) bei der FFS-Bewegung bewegt wird und zur Reduzierung der FFS-Ablenkung der FFS-Startpunkt (FS) näher zum FFS-Endpunkt (FE) verschoben wird und/oder der FFS-Endpunkt (FE) näher zum FFS-Startpunkt (FA) verschoben wird, bevorzugt wobei
- die Bewegungszeit des Fokuspunkts (F) zwischen FFS-Startpunkt (FS) und FFS-Endpunkt (FE) bei der reduzierten FFS-Ablenkung gleich der vorgegebenen FFS-Ablenkung ist oder
- die Geschwindigkeit des Fokuspunkts (F) zwischen FFS-Startpunkt (FS) und FFS-Endpunkt (FE) bei der reduzierten FFS-Ablenkung gleich der vorgegebenen FFS-Ablenkung ist und eine FFS-Ablenkung erst nach einer vorgegebenen Wartezeit nach Beginn der betreffenden Aufnahme und/oder mit einer Pausezeit zum Ende der Aufnahme erfolgt,
bevorzugt wobei der FFS-Startpunkt (FS) bei einem Winkel zur einen Seite der Senkrechten über dem Detektor im Wesentlichen dem FFS-Endpunkt (FE) bei dem entsprechenden Winkel zur anderen Seite der Senkrechten entspricht und umgekehrt.

4. Verfahren nach einem der vorangehenden Ansprüche, wobei in dem Fall, dass eine Überstrahlung vorliegt, Strahl-Steuerdaten (DS) zur Reduzierung der Intensität des Röntgenstrahls für diese Aufnahme dem Steuerdatensatz (D) hinzugefügt werden.

5. Verfahren nach einem der vorangehenden Ansprüche, wobei der Steuerdatensatz (D) zusätzlich Blenden-Steuerdaten (DB) zum Nachführen der Kollimatorblende (C) des Röntgenstrahlers (4) entsprechend der FFS-Ablenkung des (F) Fokuspunkts umfasst,
wobei die Blenden-Steuerdaten (DB) bevorzugt dazu ausgelegt sind, die Kollimatorblende (C) in Form von zwei Bewegungen zu bewegen:
- eine Rückführungsbewegung in Richtung der kreisförmigen Bewegung des Röntgenstrahlers (4) bei ausgeschaltetem Röntgenstrahl (R), wobei die Kollimatorblende (C) nach dem Ausschalten des Röntgenstrahls (R) an eine Startposition relativ zum Röntgenstrahler (4) bewegt wird,
- eine kontinuierliche Nachführung gegen die Richtung der kreisförmigen Bewegung des Röntgenstrahlers (4) bei angeschaltetem Röntgenstrahl (R), wobei die Kollimatorblende (C) von der Startposition aus bis zu einer Endposition nachgeführt wird, bevorzugt wobei die Kollimatorblende (C) so nachgeführt wird, dass sie im Wesentlichen eine konstante Position zu einer Linie zwischen Fokuspunkt (F) und einem vorbestimmten Punkt auf dem Detektor (5) hat, bevorzugt zum Mittelpunkt des Detektors (D).

6. Verfahren nach Anspruch 5, wobei die Kollimatorblende (C) während ihrer kontinuierlichen Nachführung so bewegt wird, dass sie während der Bewegung des Röntgenstrahlers (4) relativ
- zum Detektor (5) und/oder
- zu einem Fokuspunkt (F) und/oder
- zu einem Schnittpunkt einer Linie zwischen Fokuspunkt (F) und einem vorbestimmten Punkt auf dem Detektor (5) mit einer Ebene der Kollimatorblende (C)
ruht oder sich langsamer als die Bahngeschwindigkeit der kreisförmigen Bewegung in Richtung oder entgegen der kreisförmigen Bewegung bewegt oder sich zeitversetzt zu der FFS-Ablenkung bewegt.

7. Verfahren nach Anspruch 5 oder 6, wobei die Kollimatorblende (C) nur dann kontinuierlich nachgeführt wird, wenn eine FFS-Ablenkung erfolgt, insbesondere nur dann, wenn ein Strom durch die FFS-Ablenkspule (14) fließt und/oder wenn der Röntgenstrahler (4) angeschaltet ist.

8. Verfahren nach einem der Ansprüche 5 bis 7, wobei eine Änderung einer Blendenöffnung der Kollimatorblende (C) erfolgt, bevorzugt wobei die Blendenöffnung kleiner ist, je weiter der Winkel des Röntgenstrahlers (4) von der Senkrechten zum Detektor (5) abweicht.

9. Verfahren nach einem der vorangehenden Ansprüche, umfassend die zusätzlichen Schritte:
- Anfertigen einer Vielzahl von Projektionsbildern (P), wobei der Röntgenstrahl (R) des Röntgenstrahlers (4) während dessen kreisförmiger Bewegung vielfach an und ausgeschaltet wird,
- FFS-Ablenkung des Fokuspunkts (F) während der Anfertigung der Projektionsaufnahmen (P) gemäß dem Steuerdatensatz (D), bevorzugt wobei der Fokuspunkt (F) im Falle einer FFS-Ablenkung entgegen der kreisförmigen Bewegung des Röntgenstrahlers (4) geführt wird.

10. Verfahren nach Anspruch 9, wobei nach dem Anfertigen der Projektionsbilder (P) eine Rekonstruktion einer Anzahl von Bildern aus den aufgenommenen Projektionsbildern (P) erfolgt, wobei bei der Rekonstruktion bevorzugt Daten von Projektionsbildern (P) basierend auf dem Steuerdatensatz (D) unterschiedlich gewichtet werden, insbesondere wobei eine Wichtung umso geringer ist, je größer die Reduzierung der FFS-Ablenkung war.

11. System (20) zur Steuerung eines FFS-Röntgensystems (1) mit einem Röntgenstrahler (4), welcher zum Anfertigen einer Vielzahl von Projektionsbildern (P) in einer kreisförmigen Bewegung um einen Detektor (5) herum geführt wird und ein durch eine Kollimatorblende (C) kollimierter Röntgenstrahl (R) während der Bewegung vielfach an und ausgeschaltet wird, und wobei das FFS-Röntgensystem (1) für eine FFS-Ablenkung eine FFS-Ablenkspule (14) umfasst, mit der ein Fokuspunkt (F) eines den Röntgenstrahl (R) erzeugender Elektronenstrahls (E) zwischen Kathode (K) und Anode (A) des Röntgenstrahlers (4) abgelenkt werden kann, das System (20) umfassend:
- eine Simulationseinheit (9) ausgelegt zur Simulation einer Strahlgeometrie des Röntgenstrahls (R) bei einer vorgegebenen FFS-Ablenkung auf den Detektor (5) während einer Aufnahme eines Projektionsbildes (P),
- eine Ermittlungseinheit (10) ausgelegt zur Ermittlung ob eine Überstrahlung eines vorbestimmten Bereichs um den Detektor (5) durch den simulierten Röntgenstrahl (R) vorliegt,
- eine Steuerdateneinheit (11) ausgelegt zur Generieren eines Steuerdatensatzes (D).

12. Röntgensystem (1), insbesondere ein Mammographiesystem, mit einer Steuereinrichtung (12) umfassend ein System (20) nach Anspruch 11 .

13. Computerprogrammprodukt mit einem Computerprogramm, welches direkt in eine Speichereinrichtung einer Steuereinrichtung (12) eines Röntgensystems (1) ladbar ist, mit Programmabschnitten, um alle Schritte des Verfahrens nach einem der Ansprüche 1 bis 10 auszuführen, wenn das Computerprogramm in der Steuereinrichtung (12) ausgeführt wird.

14. Computerlesbares Medium, auf welchem von einer Rechnereinheit einlesbare und ausführbare Programmabschnitte gespeichert sind, um alle Schritte des Verfahrens nach einem der Ansprüche 1 bis 10 auszuführen, wenn die Programmabschnitte von der Rechnereinheit ausgeführt werden.

## Claims

1. Method for controlling an FFS X-ray system (1) with an X-ray source (4), which is guided in a circular movement around a detector (5) to produce a plurality of projection images (P), and an X-ray beam (R) collimated by a collimator aperture (C) is turned on and off many times during the movement, and wherein, for FFS deflection, the FFS X-ray system (1) comprises an FFS deflection coil (14) with which a focal point (F) of an electron beam (E) generating the X-ray beam (R) can be deflected between the cathode (K) and anode (A) of the X-ray source (4), the method comprising the steps:
- simulating a beam geometry of the X-ray beam (R) at a specified FFS deflection onto the detector (5) during recording of a projection image (P),
- determining whether cross-radiation is present in a predetermined region around the detector (5) by the simulated X-ray beam (R),
- generating FFS control data (DF) for the recording, which, in the event of cross-radiation being present for the recording, causes FFS deflection that is reduced relative to the specified FFS deflection for this recording, and otherwise causes the specified FFS deflection,
- repeating the steps for at least one further recording,
- generating a control data set (D) comprising the generated FFS control data (DF) for controlling an FFS X-ray system (1).

2. Method according to claim 1, wherein the FFS deflection for a recording is reduced in such a way that the FFS deflection is reduced to zero for the relevant recordings or no recording of a projection image (P) takes place.

3. Method according to one of the preceding claims, wherein the specified FFS deflection has an FFS starting point (FS) and an FFS end point (FE) between which the focal point (F) is moved during the FFS movement and, to reduce the FFS deflection, the FFS starting point (FS) is shifted closer to the FFS end point (FE) and/or the FFS end point (FE) is shifted closer to the FFS starting point (FA), preferably wherein
- the movement time of the focal point (F) between the FFS starting point (FS) and the FFS end point (FE) with the reduced FFS deflection is equal to the specified FFS deflection, or
- the speed of the focal point (F) between the FFS starting point (FS) and FFS end point (FE) with the reduced FFS deflection is equal to the specified FFS deflection and FFS deflection only takes place after a specified waiting time after the start of the relevant recording and/or with a pause at the end of the recording,
preferably wherein the FFS starting point (FS) at an angle to one side of the vertical above the detector substantially corresponds to the FFS end point (FE) at the corresponding angle to the other side of the vertical and vice versa.

4. Method according to one of the preceding claims, wherein, in the event of cross-radiation being present, beam control data (DS) for reducing the intensity of the X-ray beam for this recording is added to the control data set (D).

5. Method according to one of the preceding claims, wherein the control data set (D) additionally comprises aperture control data (DB) for tracking the collimator aperture (C) of the X-ray source (4) according to the FFS deflection of the (F) focal point,
wherein the aperture control data (DB) is preferably configured to move the collimator aperture (C) in the form of two movements:
- a return movement in the direction of the circular movement of the X-ray source (4) when the X-ray beam (R) is switched off, wherein, after the X-ray beam (R) is switched off, the collimator aperture (C) is moved to a starting position relative to the X-ray source (4),
- continuous tracking against the direction of the circular movement of the X-ray source (4) when the X-ray beam (R) is switched on, wherein the collimator aperture (C) is tracked from the starting position to an end position, preferably wherein the collimator aperture (C) is tracked such that it substantially has a constant position with respect to a line between the focal point (F) and a predetermined point on the detector (5), preferably with respect to the centre of the detector (D).

6. Method according to claim 5, wherein, during its continuous tracking, the collimator aperture (C) is moved such that, during the movement of the X-ray source (4) relative
- to the detector (5) and/or
- to a focal point (F) and/or
- to a point of intersection of a line between the focal point (F) and a predetermined point on the detector (5) with a plane of the collimator aperture (C),
it is stationary or moves more slowly than the orbital speed of the circular movement toward or counter to the circular movement or moves with a time offset to the FFS deflection.

7. Method according to claim 5 or 6, wherein the collimator aperture (C) is only continuously tracked when FFS deflection takes place, in particular only when current flows through the FFS deflection coil (14) and/or when the X-ray source (4) is switched on.

8. Method according to one of claims 5 to 7, wherein a change to an aperture opening of the collimator aperture (C) takes place, preferably wherein, the further the angle of the X-ray source (4) deviates from the vertical to the detector (5), the smaller the aperture opening.

9. Method according to one of the preceding claims comprising the additional steps:
- producing a plurality of projection images (P), wherein the X-ray beam (R) of the X-ray source (4) is turned on and off many times during its circular movement,
- FFS deflection of the focal point (F) during the production of the projection recordings (P) according to the control data set (D), preferably wherein, in the event of FFS deflection, the focal point (F) is guided counter to the circular movement of the X-ray source (4).

10. Method according to claim 9, wherein, after the production of the projection images (P), a number of images are reconstructed from the recorded projection images (P), wherein, during the reconstruction, preferably data from projection images (P) is weighted differently based on the control data set (D), in particular wherein, the greater the reduction of the FFS deflection was, the lower the weighting.

11. System (20) for controlling an FFS X-ray system (1) with an X-ray source (4), which is guided in a circular movement around a detector (5) to produce a plurality of projection images (P), and an X-ray beam (R) collimated by a collimator aperture (C) is turned on and off many times during the movement, and wherein, for FFS deflection, the FFS X-ray system (1) comprises an FFS deflection coil (14) with which a focal point (F) of an electron beam (E) generating the X-ray beam (R) can be deflected between the cathode (K) and anode (A) of the X-ray source (4), the system (20) comprising:
- a simulation unit (9) configured to simulate a beam geometry of the X-ray beam (R) at a specified FFS deflection onto the detector (5) during recording of a projection image (P),
- a determining unit (10) configured to determine whether cross-radiation is present in a predetermined region around the detector (5) by the simulated X-ray beam (R),
- a control data unit (11) configured to generate a control data set (D).

12. X-ray system (1), in particular a mammography system, with a control facility (12) comprising a system (20) according to claim 11.

13. Computer program product with a computer program, which can be loaded directly into a storage facility of a control facility (12) of an X-ray system (1), with program sections for executing all steps of the method according to one of claims 1 to 10 when the computer program is executed in the control facility (12).

14. Computer-readable medium on which program sections that can be read and executed by a computer unit are stored in order to execute all steps of the method according to one of claims 1 to 10 when the program sections are executed by the computer unit.

## Revendications

1. Procédé de commande d'un système (1) à rayons X FFS comprenant un émetteur (4) de rayons X, que, pour la production d'une pluralité d'images (P) de projection, on fait passer en un mouvement circulaire autour d'un détecteur (5) et on projette et on arrête, plusieurs fois pendant le mouvement, un faisceau (R) de rayons X collimaté par un diaphragme (C) formant collimateur, et dans lequel le système (1) à rayons X FFS comprend, pour une déviation FFS, une bobine (14) de déviation FFS, par laquelle on peut dévier un point (F) focal d'un faisceau (E) d'électrons produisant le faisceau (R) de rayons X entre une cathode (K) et une anode (A) de l'émetteur (4) de rayons X, le procédé comprenant les stades :
- simulation d'une géométrie du faisceau (R) de rayons X, lors d'une déviation FFS donnée à l'avance sur le détecteur (5) pendant un enregistrement d'une image (P) de projection,
- détermination, s'il y a une surexposition au rayonnement, d'une partie déterminée à l'avance autour du détecteur (5) par le faisceau (R) de rayons X simulé,
- création de données (DF) de commande FFS pour l'enregistrement, qui, dans le cas où il y a pour l'enregistrement une surexposition au rayonnement, provoque, pour cet enregistrement, une déviation FFS réduite par rapport à la déviation FFS donnée à l'avance et sinon provoque la déviation FFS donnée à l'avance,
- répétition des stades pour au moins un autre enregistrement,
- création d'un ensemble (D) de données de commande comprenant les données (DF) de commande FFS créées pour la commande d'un système (1) de rayons X FFS.

2. Procédé suivant la revendication 1, dans lequel une réduction de la déviation FFS pour un enregistrement a lieu, à tel point que, pour les enregistrements concernés, la déviation FFS est réduite à zéro ou qu'il n'y a pas d'enregistrement d'une image (P) de projection.

3. Procédé suivant l'une des revendications précédentes, dans lequel la déviation FFS donnée à l'avance a un point (FS) de début FFS et un point (FE) de fin FFS, entre lesquelles on déplace le point (F) focal lors du déplacement FFS et on décale, pour la réduction de la déviation FFS, le point (FS) de début FFS en le rapprochant (FE) de fin FFS et/ou on décale le point (FE) de fin FFS en le rapprochant du point (FA) de début FFS, dans lequel de préférence
- le temps de déplacement du point (F) focal entre le point (FS) de début FFS et le point (FE) de fin FFS, lors de la déviation FFS réduite, est égale à la déviation FFS donnée à l'avance ou
- la vitesse du point (F) focal entre le point (FS) de début FFS et le point (FE) de fin FFS, lors de la déviation FFS réduite, est égale à la déviation FFS donnée à l'avance et il se produit une déviation FFS seulement après un temps d'attente donné à l'avance après le début de l'enregistrement concerné et/ou avec un temps de pause à la fin de l'enregistrement,
dans lequel de préférence le point (FS) de début FFS correspond, pour un angle par rapport à un côté de la verticale au-dessus du détecteur, sensiblement au point (FE) de fin FFS pour l'angle correspondant par rapport à l'autre côté de la verticale et inversement.

4. Procédé suivant l'une des revendications précédentes, dans lequel, dans le cas où il y a une surexposition au rayonnement, on ajoute à l'ensemble (D) de données de commande, des données (DS) de commande de faisceau pour la réduction de l'intensité du faisceau de rayons X pour cet enregistrement.

5. Procédé suivant l'une des revendications précédentes, dans lequel l'ensemble (D) de données de commande comprend supplémentairement des données (DB) de commande de diaphragme pour suivre le diaphragme (C) formant collimateur de l'émetteur (4) de rayons X conformément à la déviation FFS du point (F) focal, dans lequel on conçoit les données (DB) de commande de diaphragme de préférence pour déplacer le diaphragme (C) formant collimateur sous la forme de deux mouvements :
- un mouvement de retour dans le sens du mouvement circulaire de l'émetteur (4) de rayons X alors que le faisceau (R) de rayons X n'est pas projeté, dans lequel, après l'arrêt du faisceau (R) de rayons X, on déplace le diaphragme (C) formant collimateur en l'amenant à une position de début par rapport à l'émetteur (4) de rayons X,
- un suivi continu dans le sens inverse du mouvement circulaire de l'émetteur (4) de rayons X, alors que le faisceau (R) de rayons X est projeté, dans lequel on suit le diaphragme (C) formant collimateur de la position de début jusqu'à une position de fin, dans lequel de préférence on suit le diaphragme (C) formant collimateur, de manière à ce qu'il ait sensiblement une position constante par rapport à une ligne entre le point (F) focal et un point déterminé à l'avance sur le détecteur (5), de préférence par rapport à un centre du détecteur (D).

6. Procédé suivant la revendication 5, dans lequel on déplace le diaphragme (C) formant collimateur pendant son suivi continu, de manière à ce qu'il repose par un plan du diaphragme (C) formant collimateur, pendant le mouvement de l'émetteur (4) de rayons X relativement
- au détecteur (5) et/ou
- à un point (F) focal et/ou
- à un point d'intersection d'une ligne entre le point (F) focal
et un point déterminé à l'avance sur le détecteur (5) ou de manière à ce qu'il se déplace plus lentement que la vitesse de trajectoire du mouvement circulaire dans le sens du mouvement circulaire ou en sens inverse ou se déplace de manière décalée par rapport à la déviation FFS.

7. Procédé suivant la revendication 5 ou 6, dans lequel on ne suit en continu le diaphragme (C) formant collimateur, que s'il y a une déviation FFS, en particulier que s'il passe un courant dans la bobine (14) de déviation FFS et/ou si l'émetteur (4) de rayons X est en projection.

8. Procédé suivant l'une des revendications 5 à 7, dans lequel une modification de l'ouverture du diaphragme (C) formant collimateur a lieu, dans lequel de préférence l'ouverture du diaphragme est d'autant plus petite que l'angle de l'émetteur (4) de rayons X s'écarte de la perpendiculaire au détecteur (5).

9. Procédé suivant l'une des revendications précédentes, comprenant les stades supplémentaires :
- production d'une pluralité d'images (P) de projection, dans lequel, pendant son mouvement circulaire, on projette et on cesse de projeter plusieurs fois le faisceau (R) de l'émetteur (4) de rayons X,
- déviation FFS du point (F) focal pendant la production des enregistrements (P) de projection suivant l'ensemble (D) de données de commande, dans lequel de préférence on fait passer le point (F) focal dans le cas d'une déviation FFS dans le sens contraire au mouvement circulaire de l'émetteur (4) de rayons X.

10. Procédé suivant la revendication 9, dans lequel après la production des images (P) de projection, a lieu une reconstruction d'un certain nombre d'images, à partir des images (P) de projection enregistrées, dans lequel, lors de la reconstruction, de préférence, on pondère différemment des données d'image (P) de projection reposant sur l'ensemble (D) de données d'image, dans lequel, en particulier, une pondération est d'autant plus petite que la réduction de la déviation FFS était plus grande.

11. Système (20) de commande d'un système (1) de rayons X FFS, comprenant un émetteur (4) de rayons X, que, pour la production d'une pluralité d'images (P) de projection, on fait passer en un mouvement circulaire autour d'un détecteur (5) et on projette et on arrête, plusieurs fois pendant le mouvement, un faisceau (R) de rayons X collimaté par un diaphragme (C) formant collimateur, et dans lequel le système (1) à rayons X FFS comprend, pour une déviation FFS, une bobine (14) de déviation FFS, par laquelle on peut dévier un point (F) focal d'un faisceau (E) d'électrons produisant le faisceau (R) de rayons X entre une cathode (K) et une anode (A) de l'émetteur (4) de rayons X, le système (20) comprenant :
- une unité (9) de simulation conçue pour la simulation d'une géométrie du faisceau (R) de rayons X pour une déviation FFS donnée à l'avance sur le détecteur (5) pendant l'enregistrement d'une image (P) de projection,
- une unité (10) de détermination conçue pour déterminer s'il y a une surexposition au rayonnement d'une partie déterminée à l'avance autour du détecteur (5) par le faisceau (R) de rayons X simulé,
- une unité (11) de données de commande conçue pour créer un ensemble (D) de données de commande.

12. Système (1) à rayons X, en particulier système de mammographie, comprenant un dispositif (12) de commande comprenant un système (20) suivant la revendication 11.

13. Produit de programme d'ordinateur, ayant un programme d'ordinateur, qui peut être chargé directement dans un dispositif de mémoire d'un dispositif (12) de commande d'un système (1) à rayons X, comprenant des parties de programme pour exécuter tous les stades du procédé suivant l'une des revendications 1 à 10, lorsque le programme d'ordinateur est exécuté dans le dispositif (12) de commande.

14. Support déchiffrable par ordinateur, sur lequel sont mises en mémoire des parties de programme pouvant être déchiffrées et exécutées par une unité informatique, afin d'exécuter tous les stades du procédé suivant l'une des revendications 1 à 10, lorsque les parties de programme sont exécutées par l'unité informatique.
